# FASCICULE DE BREVET EUROPEEN

(11) **EP 2 901 369 B1**
(45) Date de publication et mention de la délivrance du brevet: **29.06.2016**
(21) Numéro de dépôt: 14730817.5
(22) Date de dépôt: 05.06.2014
(51) Int. Cl.: G06K 9/00

(54) **DISPOSITIF DE CAPTURE D'UNE IMAGE REPRÉSENTATIVE D'UNE EMPREINTE D'UNE PARTIE DU CORPS D'UNE PERSONNE**
VORRICHTUNG ZUR AUFNAHME EINES BILDES MIT DEM ABDRUCK EINES KÖRPERTEILS EINER PERSON
DEVICE FOR CAPTURING AN IMAGE REPRESENTING AN IMPRESSION OF A BODY PART OF A PERSON

(30) Priorité: 07.06.2013 FR 1355256
(43) Date de publication de la demande: 05.08.2015
(73) Titulaire: Morpho, 92130 Issy-les-Moulineaux (FR)
(72) Inventeur: DUMONT, Denis, 92130 Issy Les Moulineaux (FR); RIEUL, François, 92130 Issy Les Moulineaux (FR); CRUCHAGA, Michel, 92130 Issy Les Moulineaux (FR); PICARD, Sylvaine, 92130 Issy Les Moulineaux (FR)
(74) Mandataire: Le Guen-Maillet
(86) Numéro de dépôt international: PCT/EP2014/061753
(87) Numéro de publication internationale: WO 2014/195429

(56) Documents cités:
- EP-A2- 0 901 093
- EP-A2- 1 316 913
- WO-A2-2008/052017
- US-A1- 2006 047 971
- US-A1- 2008 054 875

## Description

La présente invention concerne un dispositif de capture d'une image représentative d'une empreinte, ainsi qu'un dispositif d'identification d'une personne utilisant un tel dispositif de capture. Elle trouve application dans le domaine de la reconnaissance biométrique et en particulier dans le domaine de l'identification par analyse des empreintes d'une personne.

Actuellement, la capture d'une empreinte, en particulier digitale, consiste à placer la partie du corps, en particulier le doigt, porteur de l'empreinte sur une vitre transparente, et à capturer une image de ladite empreinte à l'aide d'une caméra à travers cette vitre. L'image ainsi capturée est ensuite comparée à des images de référence d'une base de données.

Selon l'exposition lumineuse, l'image capturée peut manquer de contraste, ce qui peut générer des erreurs lors de la comparaison.

Un objet de la présente invention est de proposer un dispositif de capture d'une empreinte qui ne présente pas les inconvénients de l'état de la technique et qui, en particulier, permet la capture d'une image avec un contraste amélioré.

A cet effet, est proposé un dispositif de capture d'une image représentative d'une empreinte d'une partie du corps d'une personne, le dispositif de capture comportant:
- une couche de base,
- une électrode de référence recouvrant la couche de base,
- des capsules électroniques fixées sous forme d'une matrice sur l'électrode de référence, chaque capsule électronique étant constituée d'une microcapsule remplie d'un milieu liquide et de particules de deux couleurs différentes et de charges électriques opposées,
- une plaque de support recouvrant les capsules électroniques et servant d'appui à ladite partie,
- une caméra destinée à prendre une image des capsules électroniques, et
- une électrode active destinée à venir en contact avec ladite partie et électriquement connectée à une source de tension délivrant une tension électrique non nulle.

Selon un mode de réalisation particulier, la caméra est au-dessus de la partie, et la plaque de support est transparente.

Selon un autre mode de réalisation particulier, la caméra est sous la couche de base, et la couche de base et l'électrode de référence sont transparentes.

Avantageusement, le dispositif de capture comporte en outre, pour chaque capsule électronique, au moins une électrode électriquement isolée des autres électrodes et insérée dans la plaque support, chaque électrode présentant une première face en contact avec ladite capsule électronique et une deuxième face orientée vers l'extérieur pour être en contact avec ladite partie.

Avantageusement, le dispositif de capture comporte en outre une plaque de réinitialisation électriquement conductrice destinée à être appliquée sur l'ensemble des capsules électroniques et soumise à une tension de signe opposée à celle de l'électrode active.

Avantageusement, la couche de base, l'électrode de référence, la plaque de support et les éventuelles électrodes sont souples.

Avantageusement, le dispositif de capture comporte un système d'éclairement destiné à éclairer les parties des capsules électroniques faisant face à la caméra.

Selon une première variante, le système d'éclairement prend la forme d'un ensemble d'au moins une source lumineuse disposée au voisinage de la caméra et éclairant la face de la couche de base en vis-à-vis.

Selon une deuxième variante, le système d'éclairement prend la forme d'un ensemble d'au moins une source lumineuse disposée le long de la tranche de la couche de base de manière à projeter un flux lumineux à l'intérieur de ladite couche de base.

Avantageusement, les particules blanches chargées positivement sont phosphorescentes.

Avantageusement, les particules blanches chargées positivement sont fluorescentes.

L'invention propose également un dispositif d'identification comportant un dispositif de capture selon l'un des modes de réalisation précédents, une base de données contenant des images d'empreintes de référence, et une unité de traitement prévue pour comparer l'image capturée par la caméra avec les images d'empreintes de référence de la base de données et pour prendre une décision quant à l'identité de la personne ayant présenté sa partie à partir des résultats des comparaisons.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
la Fig. 1 est une représentation schématique d'un dispositif de capture selon un premier mode de réalisation de l'invention,
la Fig. 2 est une représentation schématique d'un dispositif de capture selon un deuxième mode de réalisation de l'invention,
la Fig. 3 est une représentation schématique d'un dispositif de capture selon un deuxième mode de réalisation de l'invention, et
la Fig. 4 montre un exemple d'utilisation.

Dans la suite de la description, le terme "empreinte" s'applique aussi bien à une empreinte digitale, à une empreinte palmaire ou à toute autre empreinte portée par une partie du corps.

Dans la suite de la description, une "capsule électronique" est constituée d'une microcapsule qui présente de préférence un diamètre inférieur ou égal à 50 µm, qui est remplie d'un milieu liquide, de préférence de l'huile, et de particules de deux couleurs différentes, de préférences des particules blanches et des particules noires, les particules de couleurs différentes ayant des charges électriques opposées, de préférence les particules blanches sont chargées positivement et les particules noires sont chargées négativement.

De telles capsules électroniques sont par exemple utilisées par la société Eink pour réaliser ce qui est appelé une encre électronique.

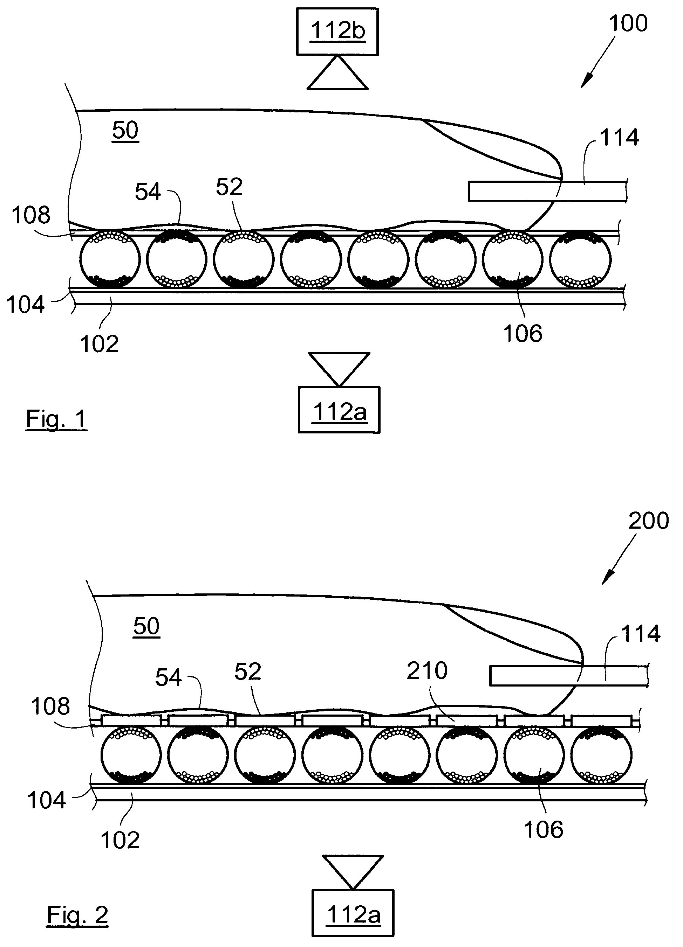

La Fig. 1 montre un dispositif de capture 100 selon un premier mode de réalisation de l'invention. Le dispositif de capture 100 est destiné à capturer une image représentative d'une empreinte portée par une partie du corps d'une personne, ici l'empreinte d'un doigt 50. L'empreinte du doigt 50 est réalisée par une série de crêtes 52 et une série de vallées 54.

Le dispositif de capture 100 comprend:
- une couche de base 102,
- une électrode de référence 104 qui recouvre la couche de base 102,
- des capsules électroniques 106 fixées sous forme d'une matrice sur l'électrode de référence 104,
- une plaque de support 108 qui recouvre les capsules électroniques 106 et qui sert d'appui au doigt 50,
- une caméra 112a, 112b destinée à prendre une image des capsules électroniques 106, et
- une électrode active 114 destinée à venir en contact avec le doigt 50 et à soumettre ledit doigt 50 à une tension électrique non nulle.

Les dimensions en lignes-colonnes de la matrice et le nombre de capsules électroniques 106 dépendent de la surface de l'empreinte.

La plaque de support 108 est électriquement non conductrice.

L'électrode active 114 est électriquement connectée à une source de tension délivrant une tension électrique non nulle.

L'électrode de référence 104 est de préférence soumise à une tension nulle.

Dans le cas de particules blanches chargées positivement et de particules noires chargées négativement, la tension appliquée au doigt 50 par l'électrode active 114 est négative et de préférence de l'ordre de -5V.

La caméra 112a, 112b peut être une caméra à capteur CCD ou CMOS ou un capteur plat (technologie FTF par exemple) située directement en dessous de la couche de base 102.

Le fonctionnement du dispositif de capture 100 est le suivant.

Le doigt 50 est posé sur la plaque de support 108 et par contact avec l'électrode active 114 est soumis à une tension.

Par contact, cette tension se transmet aux capsules électroniques 106 qui sont en contact avec le doigt 50 par l'intermédiaire des crêtes 52.

Pour chaque capsule électronique 106, les particules de la capsule électronique 106 qui portent la même charge électrique que le doigt 50 sont alors repoussées vers l'électrode de référence 104, tandis que les particules qui portent une charge électrique opposée à celle du doigt 50 sont attirées vers le doigt 50.

Une image des capsules électroniques 106 est alors capturée par la caméra 112a, 112b. Cette image est représentative des crêtes 52 et des vallées 54 de l'empreinte du doigt 50 et l'image présente alors un contraste renforcé car il est dû à la différence entre les particules de deux couleurs différentes et n'est plus dû à un éclairement du doigt 50.

Lorsque la caméra 112b est au-dessus du doigt 50, la capture de l'image nécessite d'abord le retrait du doigt 50 et la plaque de support 108 doit être transparente.

Lorsque la caméra 112a est sous la couche de base 102, la capture de l'image peut s'effectuer sans le retrait du doigt 50. Dans ce cas, la couche de base 102 et l'électrode de référence 104 sont transparentes.

La Fig. 2 montre un dispositif de capture 200 selon un deuxième mode de réalisation de l'invention. Le dispositif de capture 200 se distingue du dispositif de capture 100 du premier mode de réalisation par le fait que, pour chaque capsule électronique 106, le dispositif de capture 200 présente une électrode 210 électriquement isolée des autres électrodes 210 et insérée dans la plaque support 108.

Dans ce mode de réalisation, la caméra 112a est sous la couche de base 102, et la couche de base 102 et l'électrode de référence 104 sont transparentes.

Chaque électrode 210 présente une première face en contact avec la capsule électronique 106 associée et une deuxième face orientée vers l'extérieur pour être en contact avec le doigt 50, c'est-à-dire du côté opposé par rapport à ladite capsule électronique 106. Les deux faces sont conductrices entre elles.

La caméra 112a capture une image des capsules électroniques 106 à travers la couche de base 102 et l'électrode de référence 104.

Le fonctionnement du dispositif de capture 200 est similaire à celui du dispositif de capture 100 du premier mode de réalisation sauf que la tension à laquelle le doigt 50 est soumise se transmet aux capsules électroniques 106 à travers les électrodes 210 qui sont en contact avec le doigt 50 par l'intermédiaire des crêtes 52.

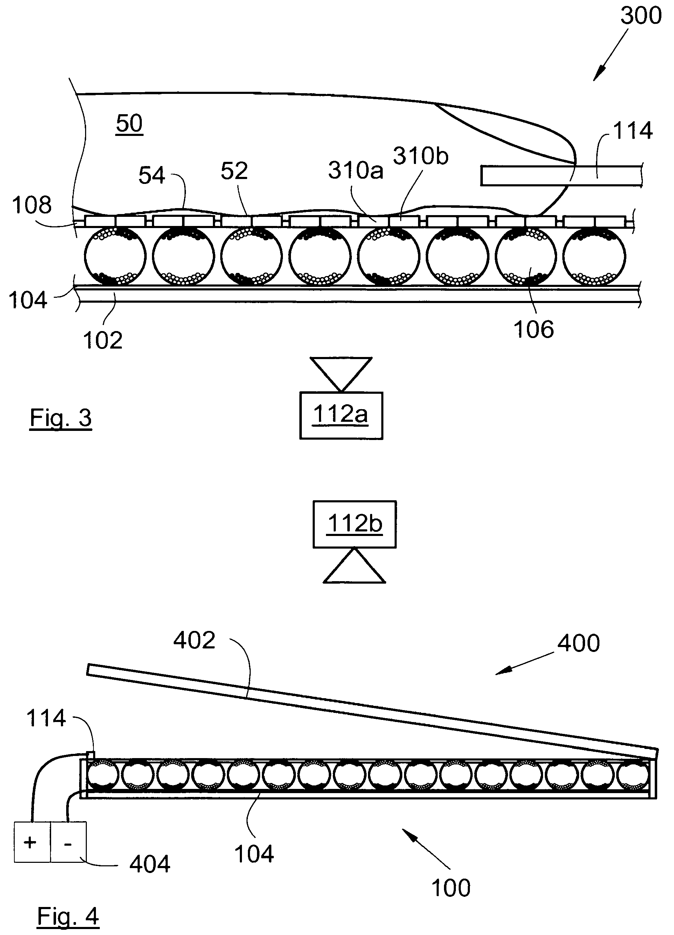

La Fig. 3 montre un dispositif de capture 300 selon un troisième mode de réalisation de l'invention. Le dispositif de capture 300 se distingue du dispositif de capture 200 du deuxième mode de réalisation par le fait que pour chaque capsule électronique 106, le dispositif de capture 300 présente deux électrodes 310a et 310b, électriquement isolées l'une de l'autre et prenant la place de l'électrode 210 unique du deuxième mode de réalisation.

Les deux électrodes 310a et 310b sont insérées dans la plaque support 108, et présentent chacune une première face en contact avec ladite capsule électronique 106 et une deuxième face orientée vers l'extérieur pour être en contact avec le doigt 50.

Ce mode de réalisation permet d'améliorer la résolution en multipliant par deux les possibilités de migration des particules.

Lorsque la caméra 112a est sous la couche de base 102, le dispositif de capture 100, 200, 300 comporte de préférence un système d'éclairement destiné à éclairer les parties des capsules électroniques 106 faisant face à la caméra 112a et donc destinées à apparaître sur l'image capturée.

Le système d'éclairement peut prendre la forme d'un ensemble d'au moins une source lumineuse, chacune étant disposée au voisinage de la caméra 112a et éclairant la face de la couche de base 102 en vis-à-vis.

Le système d'éclairement peut prendre la forme d'un ensemble d'au moins une source lumineuse, chacune étant disposée le long de la tranche de la couche de base 102 de manière à projeter un flux lumineux à l'intérieur de ladite couche de base 102 qui sert alors de guide d'onde au flux lumineux qui se propage à l'intérieur de la couche de base 102. Au cours de la propagation, un flux lumineux qui rencontre une capsule électronique 106 dont les particules noires sont du côté de la couche de base 102 est absorbé par lesdites particules noires tandis qu'un flux lumineux qui rencontre une capsule électronique 106 dont les particules blanches sont du côté de la couche de base 102 est réfléchi par lesdites particules blanches et s'extrait ainsi de la couche de base 102 pour venir éclairer la caméra 112a.

Pour améliorer le couplage, un moyen de couplage du type colle optique est disposé entre l'électrode de référence 104 et les capsules électroniques 106.

Selon un autre variante et pour tous les modes de réalisation de l'invention, les particules blanches chargées positivement sont phosphorescentes alors que les particules noires chargées négativement ne le sont pas. La phosphorescence permet d'éclairer la caméra 112a, 112b lors de la capture de l'image. La phosphorescence est réalisée par exemple à l'aide d'un dépôt phosphorescent sur les particules.

Selon une autre variante et pour tous les modes de réalisation de l'invention, les particules blanches chargées positivement sont fluorescentes alors que les particules noires chargées négativement ne le sont pas. La fluorescence permet d'éclairer la caméra 112a, 112b lors de la capture de l'image. La fluorescence est réalisée par exemple à l'aide d'un dépôt fluorescent sur les particules. Le dépôt fluorescent est rechargé à l'aide d'une source lumineuse appropriée, par exemple par un ensemble d'au moins une source lumineuse, chacune étant disposée le long de la tranche de la couche de base 102 de manière à projeter un flux lumineux à l'intérieur de ladite couche de base 102 qui sert alors de guide d'onde au flux lumineux qui se propage à l'intérieur de la couche de base 102.

Un dispositif d'identification selon l'invention comporte un dispositif de capture 100, 200, 300 selon l'un des modes de réalisation précédents, une base de données contenant des images d'empreintes de référence, et une unité de traitement prévue pour comparer l'image capturée par la caméra 112a, 112b avec les images d'empreinte de référence de la base de données et pour prendre une décision quant à l'identité de la personne ayant présenté son doigt 50 à partir des résultats des comparaisons.

Pour effectuer une réinitialisation des capsules électroniques 106, une plaque de réinitialisation électriquement conductrice est appliquée sur l'ensemble des capsules électroniques 106 à la place du doigt 50, et cette plaque est soumise à une tension de signe opposée à celle de l'électrode active 114.

Pour effectuer une capture d'une grande surface du doigt 50, le dispositif de capture 100, 200, 300 est souple afin de pouvoir s'enrouler autour du doigt 50. Ainsi, la couche de base 102, l'électrode de référence 104, la plaque de support 108 et les éventuelles électrodes 210, 310a, 310b sont souples.

La Fig. 4 montre un système mobile 400 comportant un dispositif de capture 100 sur lequel est montée pivotante une plaque de réinitialisation 402, et une source de tension 404, par exemple une pile, dont la borne négative est connectée à l'électrode de référence 104 et dont la borne positive est connectée à l'électrode active 114.

Pour la réinitialisation, les tensions sont inversées et la plaque de réinitialisation 402 se plaque contre les capsules électroniques 106 en se fermant.

La caméra 112b peut être par exemple un téléphone portable muni d'un appareil photo qui après capture de l'image des capsules électroniques 106 transmet l'image vers la base de données des empreintes de référence.

Le système mobile 400 est cerclé d'un contact métallique et présente une forme rectangulaire avec des dimensions par exemple de l'ordre de 8 cm sur 10 cm.

Bien entendu, la présente invention n'est pas limitée aux exemples et modes de réalisation décrits et représentés, mais elle est susceptible de nombreuses variantes accessibles à l'homme du métier.

## Revendications

1. Dispositif de capture (100, 200, 300) d'une image représentative d'une empreinte d'une partie (50) du corps d'une personne, le dispositif de capture (100, 200, 300) comportant:
- une couche de base (102),
- une électrode de référence (104) recouvrant la couche de base (102),
- des capsules électroniques (106) fixées sous forme d'une matrice sur l'électrode de référence (104), chaque capsule électronique (106) étant constituée d'une microcapsule remplie d'un milieu liquide et de particules de deux couleurs différentes et de charges électriques opposées,
- une plaque de support (108) recouvrant les capsules électroniques (106) et servant d'appui à ladite partie (50),
- une caméra (112a, 112b) destinée à prendre une image des capsules électroniques (106), et
- une électrode active (114) destinée à venir en contact avec ladite partie (50) et électriquement connectée à une source de tension délivrant une tension électrique non nulle.

2. Dispositif de capture (100) selon la revendication 1, **caractérisé en ce que** la caméra (112b) est au-dessus de la partie (50), et **en ce que** la plaque de support (108) est transparente.

3. Dispositif de capture (100) selon la revendication 1, **caractérisé en ce que** la caméra (112a) est sous la couche de base (102), et **en ce que** la couche de base (102) et l'électrode de référence (104) sont transparentes.

4. Dispositif de capture (100) selon la revendication 3, **caractérisé en ce qu'**il comporte en outre, pour chaque capsule électronique (106), au moins une électrode (210, 310a, 310b) électriquement isolée des autres électrodes (210, 310a, 310b) et insérée dans la plaque support (108), chaque électrode (210, 310a, 310b) présentant une première face en contact avec ladite capsule électronique (106) et une deuxième face orientée vers l'extérieur pour être en contact avec ladite partie (50).

5. Dispositif de capture (100, 200, 300) selon l'une des revendications 1 à 4, **caractérisé en ce qu'**il comporte en outre une plaque de réinitialisation électriquement conductrice destinée à être appliquée sur l'ensemble des capsules électroniques (106) et soumise à une tension de signe opposée à celle de l'électrode active (114).

6. Dispositif de capture (100, 200, 300) selon l'une des revendications 1 à 5, **caractérisé en ce que** la couche de base (102), l'électrode de référence (104), la plaque de support (108) et les éventuelles électrodes (210, 310a, 310b) sont souples.

7. Dispositif de capture (100, 200, 300) selon l'une des revendications 1 à 6, **caractérisé en ce qu'**il comporte un système d'éclairement destiné à éclairer les parties des capsules électroniques (106) faisant face à la caméra (112a).

8. Dispositif de capture (100, 200, 300) selon la revendication 7, **caractérisé en ce que** le système d'éclairement prend la forme d'un ensemble d'au moins une source lumineuse disposée au voisinage de la caméra (112a) et éclairant la face de la couche de base (102) en vis-à-vis.

9. Dispositif de capture (100, 200, 300) selon la revendication 7, **caractérisé en ce que** le système d'éclairement prend la forme d'un ensemble d'au moins une source lumineuse disposée le long de la tranche de la couche de base (102) de manière à projeter un flux lumineux à l'intérieur de ladite couche de base (102).

10. Dispositif de capture (100, 200, 300) selon l'une des revendications 1 à 6, **caractérisé en ce que** les particules blanches chargées positivement sont phosphorescentes.

11. Dispositif de capture (100, 200) selon l'une des revendications 1 à 6, **caractérisé en ce que** les particules blanches chargées positivement sont fluorescentes.

12. Dispositif d'identification comportant un dispositif de capture (100, 200, 300) selon l'une des revendications précédentes, une base de données contenant des images d'empreintes de référence, et une unité de traitement prévue pour comparer l'image capturée par la caméra (112a, 112b) avec les images d'empreintes de référence de la base de données et pour prendre une décision quant à l'identité de la personne ayant présenté sa partie (50) à partir des résultats des comparaisons.

## Patentansprüche

1. Vorrichtung (100, 200, 300) zum Aufnehmen eines Bildes, das einen Abdruck eines Teils (50) des Körpers einer Person darstellt, wobei die Aufnahmevorrichtung (100, 200, 300) Folgendes umfasst:
- eine Basisschicht (102),
- eine Referenzelektrode (104), die die Basisschicht (102) abdeckt,
- elektronische Kapseln (106), die in Form einer Matrix auf der Referenzelektrode (104) befestigt sind, wobei jede elektronische Kapsel (106) aus einer Mikrokapsel gebildet ist, die mit einem flüssigen Medium und mit Partikeln mit zwei verschiedenen Farben und entgegengesetzten elektrischen Ladungen gefüllt ist,
- eine Trägerplatte (108), die die elektronischen Kapseln (106) abdeckt und als Abstützung für den Teil (50) dient,
- eine Kamera (112a, 112b), die dazu bestimmt ist, ein Bild der elektronischen Kapseln (106) aufzunehmen, und
- eine aktive Elektrode (114), die dazu bestimmt ist, mit dem Teil (50) in Kontakt zu gelangen und mit einer Spannungsquelle, die eine von null verschiedene elektrische Spannung liefert, elektrisch verbunden zu werden.

2. Aufnahmevorrichtung (100) nach Anspruch 1, **dadurch gekennzeichnet, dass** sich die Kamera (112b) über dem Teil (50) befindet und dass die Trägerplatte (108) lichtdurchlässig ist.

3. Aufnahmevorrichtung (100) nach Anspruch 1, **dadurch gekennzeichnet, dass** sich die Kamera (112a) unter der Basisschicht (102) befindet und dass die Basisschicht (102) und die Referenzelektrode (104) lichtdurchlässig sind.

4. Aufnahmevorrichtung (100) nach Anspruch 3, **dadurch gekennzeichnet, dass** sie außerdem für jede elektronische Kapsel (106) wenigstens eine Elektrode (210, 310a, 310b) umfasst, die von anderen Elektroden (210, 310a, 310b) elektrisch isoliert ist und in die Trägerplatte (108) eingesetzt ist, wobei jede Elektrode (210, 310a, 310b) eine erste Fläche, die mit der elektronischen Kapsel (106) in Kontakt ist, und eine zweite Fläche, die nach außen orientiert ist, um mit dem Teil (50) in Kontakt zu sein, aufweist.

5. Aufnahmevorrichtung (100, 200, 300) nach einem der Ansprüche 1 bis 4, **dadurch gekennzeichnet, dass** sie außerdem eine elektrisch leitende Rücksetzplatte umfasst, die dazu bestimmt ist, auf der Gesamtheit der elektronischen Kapseln (106) aufgebracht und mit einer Spannung beaufschlagt zu werden, deren Vorzeichen zu jener der aktiven Elektrode (114) entgegengesetzt ist.

6. Aufnahmevorrichtung (100, 200, 300) nach einem der Ansprüche 1 bis 5, **dadurch gekennzeichnet, dass** die Basisschicht (102), die Referenzelektrode (104), die Trägerplatte (108) und die eventuellen Elektroden 210, 310a, 310b) nachgiebig sind.

7. Aufnahmevorrichtung (100, 200, 300) nach einem der Ansprüche 1 bis 6, **dadurch gekennzeichnet, dass** sie ein Beleuchtungssystem umfasst, das dazu bestimmt ist, die Abschnitte der elektronischen Kapseln (106), die der Kamera (112a) zugewandt sind, zu beleuchten.

8. Aufnahmevorrichtung (100, 200, 300) nach Anspruch 7, **dadurch gekennzeichnet, dass** das Beleuchtungssystem die Form einer Anordnung aus wenigstens einer Lichtquelle hat, die in der Umgebung der Kamera (112a) angeordnet ist und die gegenüber befindliche Fläche der Basisschicht (102) beleuchtet.

9. Aufnahmevorrichtung (100, 200, 300) nach Anspruch 7, **dadurch gekennzeichnet, dass** das Beleuchtungssystem die Form einer Anordnung aus wenigstens einer Lichtquelle hat, die längs des Randes der Basisschicht (102) angeordnet ist, derart, dass ein Lichtstrom in die Basisschicht (102) projiziert wird.

10. Aufnahmevorrichtung (100, 200, 300) nach einem der Ansprüche 1 bis 6, **dadurch gekennzeichnet, dass** die positiv geladenen weißen Partikel phosphoreszierend sind.

11. Aufnahmevorrichtung (100, 200) nach einem der Ansprüche 1 bis 6, **dadurch gekennzeichnet, dass** die positiv geladenen weißen Partikel fluoreszierend sind.

12. Identifizierungsvorrichtung, die eine Aufnahmevorrichtung (100, 200, 300) nach einem der vorhergehenden Ansprüche, eine Datenbank, die Bilder von Referenzabdrücken enthält, und eine Verarbeitungseinheit, die dazu vorgesehen ist, das von der Kamera (112a, 112b) aufgenommene Bild mit den Bildern von Referenzabdrücken der Datenbank zu vergleichen und eine Entscheidung hinsichtlich der Identität der Person, die ihren Teil (50) präsentiert hat, anhand der Vergleichsergebnisse zu treffen, umfasst.

## Claims

1. Device for capturing (100, 200, 300) an image representing a print of a part (50) of the body of a person, the capture device (100, 200, 300) comprising:
- a base layer (102),
- a reference electrode (104) covering the base layer (102),
- electronic capsules (106) fixed in the form of a matrix on the reference electrode (104), each electronic capsule (106) consisting of a microcapsule filled with a liquid medium and particles with two different colours and opposite electrical charges,
- a support plate (108) covering the electronic capsules (106) and serving as a support for said part (50),
- a camera (112a, 112b) intended to take an image of the electronic capsules (106), and
- an active electrode (114) intended to come into contact with said part (50) and electrically connected to a voltage source delivering a non-zero electrical voltage.

2. Capture device (100) according to claim 1, **characterised in that** the camera (112b) is above the part (50), and **in that** the support plate (108) is transparent.

3. Capture device (100) according to claim 1, **characterised in that** the camera (112a) is under the base layer (102), and **in that** the base layer (102) and the reference electrode (104) are transparent.

4. Capture device (100) according to claim 3, **characterised in that** it further comprises, for each electronic capsule (106), at least one electrode (210, 310a, 310b) electrically insulated from the other electrodes (210, 310a, 310b) and inserted in the support plate (108), each electrode (210, 310a, 310b) having a first face in contact with said electronic capsule (106) and a second face oriented outwards so as to be in contact with said part (50).

5. Capture device (100, 200, 300) according to one of claims 1 to 4, **characterised in that** it further comprises an electrically conductive reinitialisation plate intended to be applied to all the electronic capsules (106) and subjected to a voltage of opposite sign to that of the active electrode (114).

6. Capture device (100, 200, 300) according to one of claims 1 to 5, **characterised in that** the base layer (102), the reference electrode (104), the support plate (108) and any electrodes (210, 310a, 310b) are flexible.

7. Capture device (100, 200, 300) according to one of claims 1 to 6, **characterised in that** it comprises an illumination system intended to illuminate the parts of the electronic capsules (106) facing the camera (112a).

8. Capture device (100, 200, 300) according to claim 7, **characterised in that** the illumination system takes the form of a set of at least one light source disposed in the vicinity of the camera (112a) and illuminating the face of the base layer (102) opposite.

9. Capture device (100, 200, 300) according to claim 7, **characterised in that** the illumination system takes the form of a set of at least one light source disposed along the edge of the base layer (102) so as to project a light flux inside said base layer (102).

10. Capture device (100, 200, 300) according to one of claims 1 to 6, **characterised in that** the positively charged white particles are phosphorescent.

11. Capture device (100, 200, 300) according to one of claims 1 to 6, **characterised in that** the positively charged white particles are fluorescent.

12. Identification device comprising a capture device (100, 200, 300) according to one of the preceding claims, a database containing reference print images, and a processing unit provided for comparing the image captured by the camera (112a, 112b) with the reference print images in the database and for taking a decision as to the identity of the person who presented his part (50) from the results of the comparisons.
